# EUROPEAN PATENT SPECIFICATION

(11) **EP 2 923 201 B1**
(45) Date of publication and mention of the grant of the patent: **16.06.2021**
(21) Application number: 13799174.1
(22) Date of filing: 21.11.2013
(51) Int. Cl.: G01N 33/14

(54) **ELECTROANALYTICAL METHODS FOR PREDICTING THE OXIDABILITY OF A WINE OR A GRAPE MUST**
ELEKTROANALYTISCHE VERFAHREN ZUR VORHERSAGE DER OXIDIERBARKEIT VON WEIN ODER TRAUBENMOST
PROCÉDÉS ÉLECTROANALYTIQUES POUR PRÉVOIR L'OXYDABILITÉ D'UN VIN OU D'UN MOÛT

(30) Priority: 21.11.2012 US 201261729154 P
(43) Date of publication of application: 30.09.2015
(73) Proprietor: Vinventions USA, LLC, Zebulon, NC 27597 (US)
(72) Inventor: UGLIANO, Maurizio, 84000 Avignon (FR); DIEVAL, Jean-Baptiste, 84100 Orange (FR); VIDAL, Stephane, 30131 Pujaut (FR); TACCHINI, Philippe, 1202 Geneva (CH)
(74) Representative: Cohausz & Florack
(86) International application number: PCT/US2013/071242
(87) International publication number: WO 2014/081936

(56) References cited:
- WO-A1-2006/094529
- ASTRIDE RODRIGUES ET AL: "Resistance to Oxidation of White Wines Assessed by Voltammetric Means", JOURNAL OF AGRICULTURAL AND FOOD CHEMISTRY, vol. 55, no. 26, 1 December 2007 (2007-12-01), pages 10557-10562, XP055094472, ISSN: 0021-8561, DOI: 10.1021/jf0723099
- ÁNGELES M. ALONSO ET AL: "Determination of Antioxidant Power of Red and White Wines by a New Electrochemical Method and Its Correlation with Polyphenolic Content", JOURNAL OF AGRICULTURAL AND FOOD CHEMISTRY, vol. 50, no. 11, 1 May 2002 (2002-05-01), pages 3112-3115, XP055095362, ISSN: 0021-8561, DOI: 10.1021/jf0116101
- PAUL A. KILMARTIN ET AL: "A Cyclic Voltammetry Method Suitable for Characterizing Antioxidant Properties of Wine and Wine Phenolics", JOURNAL OF AGRICULTURAL AND FOOD CHEMISTRY, vol. 49, no. 4, 1 April 2001 (2001-04-01), pages 1957-1965, XP055095359, ISSN: 0021-8561, DOI: 10.1021/jf001044u
- DEBORAH FRANCESCHI ET AL: "The analytical evaluation of wine oxidability", AUSTRALIAN GRAPEGROWER AND WINEMAKER, RYAN PUBLICATIONS, ADELAIDE, AU, no. 505, 1 February 2006 (2006-02-01), pages 47-52, XP008166450, ISSN: 0727-3606

## Description

### PRIORITY APPLICATION

The present application claims priority to U.S. Provisional Patent Application Serial No. 61/729,154 filed on November 21, 2012 entitled "ELECTROANALYTICAL METHOD FOR PREDICTING THE OXIDABILITY OF A WINE OR A GRAPE MUST,".

### FIELD OF DISCLOSURE

The present disclosure relates to an electroanalytical method for predicting the oxidability of a wine or a grape must. Moreover, the present disclosure relates to markers for predicting the oxidability of a wine or a grape must and the use of electrochemistry, including, but not limited to voltammetry, for predicting the oxidability of a wine or a grape must. The present disclosure also relates to a method for predicting the optimal total oxygen supply for storing a wine or a grape must in a container, to a method for wine maturation and/or ageing and to a method for selecting an optimal closure for storing a wine or a grape must in a container.

### BACKGROUND

Empirical knowledge in the traditional art of wine making combined with state-of the art research has established that oxygen plays a critical role during all stages of the wine making process, i.e. from the preparation, handling and fermentation of the grape must, to wine maturation, the bottling process and subsequent ageing of the wine. Utmost care and attention to detail must be applied during the vinification, bottling and storage process, as improper control of oxygen, including exposure to too much oxygen (oxidation) or too little oxygen (reduction), particularly during wine maturation and ageing can cause wine faults and spoil a wine's character.

It is estimated that 6 % of all wines are negatively impacted by wine faults. More than 50 % of these faults, primarily oxidation and reduction, are related to oxygen mismanagement.

From one wine to another, oxygen-needs vary considerably. If certain types of wines are starved of oxygen for longer periods of time, wine reduction may give rise to malodorous sulfur compounds such as certain sulphides, thiols and mercaptans. These cause olfactory defects which are sometimes referred to as reduced character. Even at low concentrations, these odors may completely ruin a wine's character.

Many wines, in particular white wines, are very sensitive to oxygen exposure. In these wines, which are usually meant to be consumed young, excess oxygen exposure may impair their desired fresh and fruity appeal.

For other particular types of wines, such as premium class red wines, a certain amount of oxidation is necessary to ensure full maturation of the wine flavor characteristics and prevent the formation of unpleasant aromas.

Therefore, a winemaker must strike a delicate balance between providing enough oxygen to avoid reduction and ensure full maturation of all desired wine characteristics, while taking care not to expose the wine to too much oxygen, which may lead to oxidation-mediated wine deterioration.

The vulnerability of a given wine or grape must to oxidation will primarily be defined by its intrinsic properties such as the wine's or grape must's unique chemical composition and in particular its pH and level of antioxidants.

Factors influencing the chemical composition of a wine are diverse and include the type of wine, e.g. white or red wine, the varietal used, vintage-specific factors such as climatic and soil conditions during growth of the grapes, harvesting time point and conditions, how much oxygen the grapes and grape must were already exposed to during preparation and handling, to name a few.

Winemakers have become increasingly aware of the fact that careful oxygen management throughout the winemaking process and well-reasoned wine closure selection are key determinants for an optimal development of a given wine's aroma, flavor, structure, and color. For example, A. Rodrigues et al. describe in "Resistance to Oxidation of White Wines Assessed by Voltammetric Means" in J. Agric. Food Chem. 2007, 55, 10557-10562 a methodology suited to measure the resistance to oxidation of white wines by cyclic voltammetry. The voltammetric responses of several white wines of different origin and age were analyzed in the oxidation potential range (0.2 - 1.2 V vs SCE). Current measured at fixed potentials were correlated to the concentration of ascorbic acid, S02, and total phenolics. A forced degradation study was monitored by cyclic voltammetry; from plots of current versus time, the consumption rates of oxidizable species in wine were estimated.

Once a winemaker knows exactly how much oxygen the wine in question shall be exposed to during vinification, wine maturation and ageing, state-of-the-art oxygen management equipment and closure technology allow him to conveniently set, adjust and control the oxygen exposure in every step of the winemaking process.

However, the initial estimation, how much oxygen a particular wine needs in order to develop all its desired characteristics, still remains problematic and is far from routine. Yet, this assessment is decisive, as it forms the corner stone for the subsequent oxygen management approach during the winemaking process. Thus far, the initial estimation of the oxygen-needs of a particular wine required the winemaker to have in-depth empirical knowledge and years of experience with vinification of the particular wine varietal in question. Even then, the optimal amount of oxygen a particular wine needs still has to be adjusted for each vintage. As reliable indicators for predicting how much oxygen a wine will need during maturation and ageing are lacking, this art usually remains a matter of trial and error.

The determination of the optimal amount of oxygen a particular wine needs is further complicated by the fact that this parameter varies considerably from one wine to another. Under the same conditions, different wines oxidize at different rates. It is therefore difficult to predict how much oxygen a given wine needs during winemaking, maturation and ageing and how much oxygen the wine can take before quality deterioration occurs.

Hence, there is a need for a reliable and easy to perform prediction method of a given wine's or grape must's oxidability.

### SUMMARY OF THE DETAILED DESCRIPTION

The present disclosure provides a convenient electroanalytical method for predicting the oxidability of a wine or a grape must. One embodiment of this method described herein comprises the following steps:
a) recording an electrochemical signal of a sample of the wine or grape must;
b) comparing an electrochemical signature of the electrochemical signal obtained in step a) with reference electrochemical signatures obtained from wines or grape musts with known oxidability; and
c) predicting the oxidability of the sample tested based an the comparison performed in step b), wherein the electrochemical signal is a processed electrochemical signal in which the primary signal is modulated by applying thereto a mathematical treatment based on a virtual electrochemical titration; wherein the comparison of the electrochemical signatures in step b) is performed by comparing the area beneath the curve of the processed electrochemical signal

Hence, the present disclosure concerns the use of an electrochemical signal, i.e. an electrochemically generated signal, recorded for a wine or a grape must as marker for predicting the oxidability of the wine or grape must. "Electrochemical signal" as used herein includes, but is not limited to a voltammogram. According to the present disclosure, electrochemistry-based technology, including, but not limited to voltammetry, is used for predicting the oxidability of a wine or a grape must.

Based on the prediction of the oxidability of a wine or a grape must, the present disclosure also provides a method for predicting the optimal total oxygen supply for storing a wine or a grape must in a container. This method comprises the steps of:
a) predicting the oxidability of the wine or grape must by the electroanalytical method described herein; and
b) predicting the optimal total oxygen supply based on the oxidability predicted in step a) and the desired properties that the wine shall have after storage.

The present disclosure also allows for a method for wine maturation and/or ageing comprising the steps of:
a) predicting the optimal total oxygen supply of the wine or grape must as described herein; and
b) storing the wine or grape must in a container over a defined period of time, wherein the oxygen level in the container and the storage time are adjusted so that the optimal total oxygen supply as determined in step a) is achieved at the end of the storage time.

Finally, another subject of the present disclosure is a method for selecting an optimal closure for storing a wine or a grape must in a container comprising the steps of:
a) predicting the oxidability of the wine or grape must by the electroanalytical method described herein; and
b) selecting a closure based on the oxidability predicted in step a), the oxygen transfer rate of the closure, the intended length of storage and the desired properties that the wine or grape must shall have upon opening the container after storage.

### BRIEF DESCRIPTION OF FIGURES

Further features and advantages of the present disclosure will emerge from the following detailed description of some of its embodiments shown by way of non-limiting examples in the accompanying drawings, in which:
Figure 1 is a cyclic voltammogram depicting electrochemical signals obtained for a set of six different white wines;
Figure 2 is a processed voltammogram based on the data shown in Figure 1;
Figure 3 is a bar chart depicting the results of an accelerated ageing test performed on the set of white wines, the corresponding voltammograms of which are shown in Figures 1 and 2; and
Figure 4 is a plot showing the correlation between the voltammogram in Figure 2 and the results of the accelerated ageing test depicted in Figure 3.

### DETAILED DESCRIPTION

The present disclosure provides an electroanalytical method for predicting the oxidability of a wine or a grape must, wherein one embodiment of the method comprises the following steps:
a) recording an electrochemical signal of a sample of the wine or grape must;
b) comparing an electrochemical signature of the electrochemical signal obtained in step a) with reference electrochemical signatures obtained from wines or grape musts with known oxidability; and
c) predicting the oxidability of the sample tested based on the comparison performed in step b), wherein the electrochemical signal is a processed electrochemical signal in which the primary signal is modulated by applying thereto a mathematical treatment based on a virtual electrochemical titration; wherein the comparison of the electrochemical signatures in step b) is performed by comparing the area beneath the curve of the processed electrochemical signal

The term "oxidability" as used herein is a parameter indicating how fast a given wine or grape must oxidizes, matures and/or ages in a given amount of time. Hence, oxidability is a measure for the susceptibility of a given wine or grape must to mature and eventually deteriorate more or less rapidly due to one or more reactions linked to oxidation.

As used herein, the term wine "maturation" refers to changes in the wine after fermentation and before bottling. As used herein, the term wine "ageing" refers to changes in the wine after bottling.

Oxidability can be measured experimentally by analyzing wine oxidation, maturation and/or ageing indicators over time. Many reactions occur during oxidation, maturation and ageing of wine that lead to significant changes in the composition of the wine. Therefore, many different indicators can be analyzed over time to determine wine oxidability experimentally.

During the process of wine maturation and ageing, the most obvious change occurs in the color of the wine.

In white wine, the color becomes goldcn, and later, can turn to brown if the wine is matured or aged too long.

In red wine, the purple and violet tints are progressively replaced by brick red colors during maturation and/or ageing. These color changes mostly stem from condensation reactions between anthocyanins and tannins, which results in the gradual loss of free anthocyanins and the formation of stable polymeric (anthocyanin tannin) pigments. As the wine matures or ages and more polymeric pigments arc formed, the color shifts from purple to brick red.

Hence, a particularly useful indicator of wine oxidation, maturation and ageing is the color change measured as increase in optical density at 420 nm, referred to as "browning", in both red and white wines.

Usually, oxidability of a wine can only be fully assessed once the ageing and maturation phase is completed. However, depending on the particular case, this may take from several months to years.

In order to save time, various accelerated ageing tests have been developed for wine, which simulate the maturation and ageing process. These tests allow assessment of oxidability of a given wine sample within one to several weeks. Accelerated ageing tests are known to the person skilled in the art and are for example described in Silva Ferreira et al. "Identification of Key Odorants Related to the Typical Aroma of Oxidation-Spoiled White Wines", J. Agric. Food Chem. 2003, 51, 1377-1381.

The oxidability of a wine is a key parameter as it allows the winemaker to extrapolate how much oxygen a given wine needs during winemaking, maturation and ageing and how much oxygen the wine can take before quality deterioration commences.

Once winemakers have experimentally determined the oxidability of a given wine, they can calculate the appropriate amount of oxygen for the desired winemaking outcome and choose oxygen management tools, bottling conditions and closure technology accordingly.

The disadvantage of the wine oxidability measurement methods described in the prior art is that they are often tedious and time consuming, as samples have to be taken progressively and a given wine oxidation, maturation and/or ageing indicator has to be monitored and analyzed over time.

The present disclosure is based on the finding that the electrochemical dataset obtained from a single time point electrochemical measurement, including, but not limited to a voltammetric measurement, of a wine or a grape must sample correlates with the long-term oxidability values that can be determined for the same sample experimentally over a time course of at least several days to weeks or months.

Hence the present disclosure provides a method for predicting the oxidability of a wine or a grape must, which requires only a single time point measurement.

The prediction of wine oxidability described in the present disclosure means that one can predict the intrinsic probability that a given wine or grape must will oxidize after a certain period and level of oxygen exposure. High predicted oxidability indicates that the wine or grape must is relatively prone to oxidation, whereas low predicted oxidability indicates a certain resistance to oxidation.

The electroanalytical method of the present disclosure comprises as first step the recording of an electrochemical signal of a sample of a wine or a grape must. The electrochemical signal can be obtained by any electroanalytical procedures including, but not limited to amperometry and voltammetry. For example, the electrochemical signal can be recorded as a voltammogram by means of voltammetry, a technique well known to the person skilled in the art. Voltammetry is the study of current as a function of the applied potential. The plot depicting the curve I = f(E) is referred to as voltammogram in the present disclosure.

According to an exemplary embodiment of the present disclosure, the electrochemical signal is recorded as voltammogram. Recording a voltammogram refers to a method in which the potential (expressed in Volts) of an electrode in contact with the wine or grape must sample is varied while the resulting current (expressed in Amperes) is measured.

Devices for recording voltammograms are known to the person skilled in the art and usually comprise at least two electrodes. The so-called working electrode is in direct contact with the analyte, applies the desired potential in a controlled way and transfers charge to and from the analyte. A second electrode acts as the other half of the cell. This reference electrode has a known potential with which the potential of the working electrode is gauged and it balances the charge added or removed by the working electrode.

According to one embodiment of the disclosure, the electrochemical signal and/or voltammogram is recorded using a device comprising a multiple electrode system with at least one working electrode, one reference electrode and one auxiliary electrode. Such a setup is realized in most of the currently used three electrode voltammetric systems and has the advantage that the auxiliary electrode balances the charge added or removed by the working electrode whereas the reference electrode solely acts as a half cell with known reduction potential.

In step b) of the electroanalytical method of the present disclosure, an electrochemical signature of the electrochemical signal obtained in step a) is compared with reference electrochemical signatures obtained from wines or grape musts with known oxidability.

The reference electrochemical signatures used in step b) as comparison in principle only need to be recorded once and can then be used as reference database for all subsequent measurements and predictions. For example, when the electroanalytical method for predicting oxidability described herein is applied to a sample of white wine, a database of reference electrochemical signatures obtained from different white wines with known oxidability values that were determined experimentally should be used in step b). If a red wine sample is analyzed, a database of reference electrochemical signatures obtained from different red wines with known oxidability values that were determined experimentally should be used.

In step c) of the electroanalytical method of the present disclosure, the oxidability of the sample tested is predicted based on the comparison performed in step b). This prediction is possible because the electrochemical signals obtained from a given wine or grape must sample was found to correlate with the corresponding oxidability value of the wine. Hence, if a standard curve of electrochemical signatures of different wines with known oxidability values is compared to an electrochemical signature of a wine with unknown oxidability, one can read off the latter oxidability from the corresponding position on the standard curve or database.

The method for predicting wine or grape must oxidability according to the present disclosure will allow winemakers to identify wines having higher oxidation risk, for which specific winemaking and packaging strategies can be adopted.

Furthermore, the electroanalytical method disclosed herein for predicting oxidability has the advantage that it docs not require costly or heavy equipment; the electrochemical unit used in combination with a computer for recording the electrochemical signal, including but not limited to a voltammogram, is portable. Moreover, with the method of the present disclosure, a single time point in situ measurement is sufficient for predicting the oxidability. According to the prior art this could only be achieved by analyzing an oxidation, maturation and/or ageing indicator progressively in a time course experiment lasting at least several days to weeks.

The electrochemical signal used in the electroanalytical method of the present disclosure is a processed electrochemical signal in which the primary signal is modulated by applying thereto a mathematical Operation, namely, a virtual titration by a mathematical operation. According to an exemplary embodiment of the disclosure, the processed electrochemical signal is a processed voltammogram.

It has been found to be particular useful for the electroanalytical method of the present disclosure when the mathematical Operation is such that the processed electrochemical signal depicts a bell-shaped curve. The advantage of such a processing Operation is that the comparison of the curves of the processed electrochemical signals is facilitated as for example characteristic values of the curves can be read off or calculated more easily.

For example, the area beneath the curve may be calculated by integrating the processed primary current signal over the applied potential.

The mathematical treatment for processing the primary electrochemical signal is based on the titration of an ideal and virtual antioxidant, i.e. on a numerical pseudo titration. Accordingly, a mathematical function representing the titration of an ideal oxidizing agent may be applied to the primary electrochemical signal to afford a processed electrochemical signal that is then used in step b) of the electroanalytical method of the present disclosure. According to an exemplary embodiment of the disclosure, the mathematical treatment for processing the electrochemical data is based on a Fermi-Dirac function which simulates a virtual electrochemical titration of a reference molecule which oxidation potential ranges from 0 to 1.5 V and which includes any monotonous decreasing dimensionless function between one and zero. Such a mathematical treatment is for example described in WO 2006/094529 A1. Electroanalytical measuring devices capable of applying such a mathematical function on the primary data obtained are commercially available under the trade name Edelscan (EDEL Therapeutics, Switzerland).

In the electroanalytical method of the present disclosure, the comparison of the electrochemical signatures in step b) is performed by comparing the area beneath the curve of the processed electrochemical signal. The area beneath the curve can be calculated by integrating the processed primary current signal over the applied potential. The sum of each oxidation current per potential increment is herein defined as the antioxidant power of the wine or grape must: it can be expressed either in electrical power units (Watt) or in any other specific unit such as an antioxidant power (AOP), Pouvoir AntiOxydant (PAOx), or in total antioxidant power (TAO), Pouvoir AntiOxydant Total (TAOx) or any other suitable unit.

According to an exemplary embodiment of the disclosure, the comparison of the electrochemical signatures in step b) is performed by comparing the antioxidant power of the wine or grape must to a database of antioxidant powers of wines or grape musts with known oxidability.

In principle, the electrochemical signal of the present disclosure can be measured using any suitable electrochemical techniques, including but not limited to a voltammetric or amperometric technique. According to an exemplary embodiment of the disclosure, the electrochemical signal is a voltammogram. In principle, such a voltammogram can be measured using any suitable voltammetric technique. Examples of possible types of voltammetry include linear sweep voltammetry, staircase voltammetry, square wave voltammetry, cyclic voltammetry, anodic or cathodic stripping voltammetry, adsorptive stripping voltammetry, alternating current voltammetry, polarography, rotated electrode voltammetry, normal or differential pulse voltammetry and/or chronoameperometry. According to a particular embodiment of the disclosure, the voltammogram is a cyclic voltammogram or a sweep voltammogram.

According to an exemplary embodiment of the disclosure, the voltammogram is a cyclic voltammogram. Using cyclic voltammetry as electrochemical technique for recording the voltammograms of the present disclosure has proven to be particularly useful for the types of samples analyzed with the method of the present disclosure, i.e. wine or grape must samples. According to an exemplary embodiment of the disclosure, the voltammogram is recorded by a cyclic voltammetry technique, wherein a sensor is used that comprises at least one working, one reference and one auxiliary electrode and wherein a predefined potential waveform is applied to the sensor while the variation of the electrochemical signal between the working and the auxiliary electrode is measured to afford a primary signal.

According to another embodiment of the disclosure, the voltammograms are recorded by square wave voltammetry. This technique provides the benefit of essentially negating the contribution to the current signal from the capacitive charging current. This is accomplished by increasing the potential stepwise, then measuring the current at the end of each potential change.

Devices for recording the voltammograms of the present disclosure depend on the type of voltammetry used and are principally known to the person skilled in the art. According to an exemplary embodiment of the present disclosure, the voltammogram is recorded using a device comprising a multiple electrode system with at least one working electrode, one reference electrode and one auxiliary electrode.

According to another embodiment, the reference and the auxiliary electrode can be combined in one electrode assuming both functions.

The device for recording the voltammograms of the present disclosure may consist of an electrochemical unit and an electrochemical sensor comprising at least one mono- or multi- surface working electrode, a potentiostat, and electronic processors for processing the primary electrochemical signal as described above and generating the final signal output. For example, if a mathematical treatment for processing the voltammetric data is used that is based on a virtual electrochemical titration as described above, then electronic processors programmed to apply such a function to the primary electrochemical signal should be comprised in the device.

According to an exemplary embodiment of the disclosure, for recording the electrochemical signal in step a), a working electrode is used with an electrode surface that is specially designed to allow a wide range and types of molecules to oxidize on its surface. This is particularly well achievable if a working electrode is used that has a composite material surface resulting from the assembly of different types of surfaces.

For example, activated carbon surfaces which are particularly well adapted for neutral hydrophilic compounds may be combined with gold ones, covered by an organic gel, which allow the oxidation of more hydrophobic molecules. In one embodiment of the present disclosure, composite material surface electrodes are used comprising conductive and electroactive particles, which may be made of any suitable conductive material, such as carbon (graphite), gold and/or platinum or a combination thereof. In general, the particles have the shape of flakes or balls, and exhibit a size of between 0.01 and 500 Pm, and more particularly between 1 and 20 Pm. Particles can also be mixed with or replaced by colloids, in which case the size ranges from 0.001 and 1 Pm. The specially designed surface may be applied to the surface of the electrode by conventional techniques such as printing.

According to an exemplary embodiment of the disclosure, a working electrode with a printed surface is used. According to one embodiment the printing technique for manufacturing the electrode is screen printing. The use of screen printing for the manufacture of electrodes has proven to be particularly useful due to the overall high performance and sensitivity of the electrodes, the low costs and the possibility to be mass-produced.

According to another embodiment the printing technique for manufacturing the electrode is inkjet printing. Inkjet printing is a very efficient manufacturing method as it wastes very little of the material that is necessary to produce the electrode surface, which further decreases the production costs.

In one embodiment of this disclosure, a modified screen or inkjet printed carbon electrode is used.

The carbon ink used for the manufacture of the electrode may be doped with nanoparticles such as TiO₂ nanoparticles.

According to another exemplary embodiment of the disclosure, an electrode is used that has a surface coating comprising TiO₂. In one embodiment, the TiO₂ coating is in the form of a nanocrystalline film. According to a particular embodiment, a TiO₂-coated glass electrode, e.g. a TiO₂-coated indium tin oxide (ITO) glass electrode, is used. This electrode can be a screen or inkjet printed electrode.

According to another particular embodiment of this disclosure, a screen or inkjet printed electrode comprising graphite flakes and/or TiO₂ aggregates, optionally further comprising a ruthenium compound as red-ox mediator, is used.

TiO₂-modified screen printed electrodes that are particularly useful for the applications described herein are can be manufactured according to J. Liu et al., "Antioxidant Sensors based on DNA-modified electrodes", Analytical Chemistry, Vol. 77, No. 23, (2006), 7687-7694 or J. Liu et al., "Antioxidant Redox Sensors based on DNA-modified carbon screen-printed electrodes", Analytical Chemistry, Vol. 78, No. 19 (2006), 6879-6884

According to yet another embodiment of this disclosure, a screen or inkjet printed carbon electrode comprising carbon nanotubes is used. The carbon nanotubes can be multi wall carbon nanotubes (MWCNTs) or single wall carbon nanotubes (SWCNTs). Such carbon nanotube modified printed electrodes can be manufactured according to W.-J. Guan et al., "Glucose biosensor based on multi-wall carbon nanotubes and screen printed carbon electrodes", Biosensors and Bioelectronics 21 (2005), 508-512.

According to another exemplary embodiment of the disclosure, an electrode is used that comprises an electrochemical biosensor. An electrochemical biosensor comprises at least one sensing element consisting of an immobilized biological material and a signal transducer converting a biochemical event into an appropriate electrical output signal. The sensing element is responsible for selective detection of the analyte and is usually immobilized on an electrode which serves as transducer. The biological material comprised in the sensing element may be selected from the group consisting of proteins (in particular enzymes, receptors and antibodies), oligo- or polynucleotides (in particular DNA and/or RNA) and intact cells.

The basic principle of an electrochemical biosensor as used according to the present disclosure is that the biochemical reaction between the immobilized biological material and the target analyte in the wine or grape must produces or consumes ions or electrons, which affects measurable electric properties of the analyzed wine or grape must sample, such as electric current or potential.

Methods suited for immobilizing the biological material forming the sensing element on an electrode surface are known to the person skilled in the art. The most commonly used immobilization techniques for construction of biosensors are physical adsorption, covalent binding, matrix entrapment and inter molecular cross-linking.

According to an exemplary embodiment of the disclosure, the electrode comprising an electrochemical biosensor is produced by screen or inkjet printing, wherein the biological material forming the sensing element is mixed into the carbon ink that is to be deposited on the electrode surface.

According to a further embodiment of the disclosure, an electrode comprising an electrochemical biosensor is used, wherein the sensing element comprises a biological material capable of sensing the antioxidant capacity of a wine or grape must. Electrochemical biosensors principally capable of sensing the antioxidant capacity of a wine or grape must are known to the skilled person and for example described in Prieto-Sim6n B, Cortina M, Campàs M, and Calas-Blanchard C (2008) "Electrochemical biosensors as a tool for antioxidant capacity assessment", Sens Actuators B: Chem 129: 459-466; Barroso MF, De-los-Santos-Alvarez N, Delerue-Matos C and Oliveira MBPP (2011) "Towards a reliable technology for antioxidant capacity and oxidative damage evaluation: electrochemical (bio)sensors", Biosens Bioelectron 30: 1-12; and Mello LD and Kubota LT (2007) "Biosensors as a tool for the antioxidant status evaluation" Talanta 72: 335-348.

According to an exemplary embodiment of the disclosure, the sensing element present in the electrochemical biosensor and responsible for sensing the antioxidant capacity of a wine or grape must is an enzyme. The enzyme can be selected from the group consisting of oxido-reductases, transferases, hydrolases, lyases, isomerases and ligases. The biosensor can be a multi-enzyme or a single-enzyme system.

According to an exemplary embodiment of the disclosure, an electrochemical biosensor based on an oxido-reductase capable of sensing the antioxidant capacity of a wine or grape must is used. According to a particular embodiment, the oxido-reductase is selected from the dass of NADP- or NAD±-dependent oxido-reductases, such as for example NAD⁺-dependent dehydrogenases.

As phenolic substances are among the main contributors to the antioxidant capacity of wine and grape must, an oxido-reductase with substrate specificity for a phenolic substance that is present in wine or grape must is used. An oxido-reductase may be used that is capable of catalyzing a reaction on a substrate selected from the group consisting of phenols, polyphenols, catechols, caffeic acid, chlorogenic acid, gallic acid and protocatechualdehyde. For example, the oxido-reductase can be selected from the group consisting of tyrosinases, laccases, peroxidases and polyphenol oxidases.

According to another exemplary embodiment of the disclosure, the electrochemical biosensor is based on an enzyme capable of sensing the D-lactate and/or acetaldehyde content in wine or grape must. In an exemplary embodiment, the enzyme is an oxido-reductase, in particular an oxido-reductase selected from the dass of NADP- or NAD⁺-dependent oxido-reductases, such as for example an NAD⁺-dependent dehydrogenase. Suitable oxido-reductases for use as biosensor sensing elements for sensing the D-lactate and acetaldehyde content in wine or grape must are lactate dehydrogenase and aldehyde dehydrogenase, respectively. Lactate dehydrogenase and aldehyde dehydrogenase based biosensors are principally known to the person skilled in the art and described for example in Avramescu, A.; Noguer, T.; Avramescu, M.; Marty, J.L. "Screen-printed biosensors for the control of wine quality based on lactate and acetaldehyde determination" Anal. Chim. Acta 2002, 458 (1), 203-213.

According to yet another exemplary embodiment of the disclosure, the electrochemical biosensors is based on an electrochemical technique, including but not limited to the use of an enzyme capable of sensing the sulfite content in wine or grape must. For example, a biosensor comprising the enzyme sulfite oxidase (SOD) and/or the electron acceptor cyctochrome c may be used in this respect.

According to a further embodiment of the disclosure, an electrochemical biosensor capable of sensing the antioxidant capacity of a wine or grape must is used, wherein the biosensor is selected from the group consisting of cytochrome c-based antioxidant biosensors, superoxide dismutase-based biosensors and DNA-based antioxidant biosensors.

In case an enzyme is used as sensing element in the biosensors according to the present disclosure, electrochemical mediators may be added as further component in order to optimize the biosensor performance in terms of sensitivity and interference minimization. Electrochemical mediators as used herein are defined as organic or inorganic molecules capable of accelerating heterogeneous electron transfer by acting as red-ox couples shuttling electrons from the active centre of the enzyme to the electrode surface. Examples of electrochemical mediators that can be used with the biosensors described in the present disclosure include Nile Blue, MB, NQSA, potassium hexacyanoferrate, BQ, DPIP, PMS, Prussian blue, TCNQ, cobalt (II) phtalocyanine and Meldola's blue precipitated with Reinecke salt (MBRS). The electrochemical mediators are incorporated into the working electrode prior to or together with enzyme immobilization.

According to another embodiment of the disclosure, an electrochemical biosensor is used comprising Carbon Nanotubes (CNTs) as additional components.

The above described specific electrode types are particularly suited for the herein described applications involving wine and/or grape must samples.

Devices and electrodes that are optimally suited for being applied in the methods of the present disclosure are for example described in WO 2006/094529 A1.

According to an exemplary embodiment of the disclosure, the electrochemical signal, including but not limited to a voltammogram, is recorded using a disposable electrode as working electrode. According to another embodiment of the disclosure the disposable electrode is for single use and the working electrode is discarded and exchanged between performing the electoranalytical method of the disclosure on different samples. According to an exemplary embodiment, the disposable electrode comprises or consists of a single-use strip.

Single use and/or disposable electrodes are particularly useful for the applications disclosed herein, as the risk of cross contamination is eliminated and there is no need for extensive cleaning and polishing of the electrodes, a time consuming step that is however critical in performing electrochemistry, in particular voltammetry, with conventional electrodes.

When using disposable, single-use electrodes, the samples can be analyzed on site and even *in situ*, without the need of any additional reagents or danger of contamination. Moreover, when using disposable, single-use electrodes, the electroanalytical method of the present disclosure can be applied repeatedly on different samples with less than 30 seconds in between measurements for changing the disposable electrode. This is particularly advantageous, as the method can be applied repeatedly on different samples allowing for a quick and direct unbiased comparison.

A further embodiment of the disclosure concerns the use of an electrochemical signal, including but not limited to a voltammogram, recorded for a wine or a grape must as marker for predicting the oxidability of the wine or grape must. The prediction is performed by the electroanalytical method described above.

In another embodiment of the disclosure, a method for predicting the optimal total oxygen supply for storing a wine or a grape must in a container is provided. This method comprises the steps of:
a) predicting the oxidability of the wine or grape must according to the electroanalytical method described above; and
b) predicting the optimal total oxygen supply based on the oxidability predicted in step a) and the desired properties that the wine shall have after storage.

The electroanalytical method of the present disclosure enables the winemaker based on the oxidability obtained thereby, to predict, in a further step, how much oxygen a given wine needs during winemaking, maturation and ageing to develop all its desired characteristics and how much oxygen the wine can take before quality deterioration occurs. This amount of oxygen is referred to as "optimal total oxygen supply" in the present disclosure.

Of course, the total oxygen supply chosen by a winemaker will be primarily governed by the style of wine he desires to obtain. However, the oxidability value obtainable by the electroanalytical prediction method of the present disclosure provides the winemaker with an estimate of the maximum amount of oxygen a given wine can take before quality deterioration occurs. In addition, based on comparisons with wines of known oxidability, known total oxygen supply during vinification, and known resulting organoleptic properties, the winemaker can classify the wine or grape must analyzed according to its oxidability value and predict the optimal total oxygen supply based on the desired properties that the wine shall finally have.

Hence, based on his experience with wines or grape musts with similar oxidability values as the wine or grape must in question, the winemaker can estimate and/or gauge the optimal total oxygen supply. Practical experiments have shown that the oxidability value that can be predicted with the electroanalytical method of the present disclosure is an important tool for predicting the optimal total oxygen supply.

In yet another embodiment of the disclosure, a method for wine maturation and/or ageing is provided, which comprises the steps of:
a) predicting the optimal total oxygen supply of the wine or grape must according to the electroanalytical method described above; and
b) storing the wine or grape must in a container over a defined period of time, wherein the oxygen level in the container and the storage time are adjusted so that the optimal total oxygen supply is achieved at the end of the storage time.

The container can for example be selected from the group consisting of barrel, tank, bottle, canister, jerry can and plastic bag.

In an exemplary embodiment, the optimal total oxygen supply is determined by the method described above.

Once the oxidability and optimal total oxygen supply have been determined, the winemaker can adjust and control the oxygen exposure in every step of the winemaking process so that the optimal total oxygen supply is achieved at the end of the storage time. Storage time and optimal oxygen level are interrelated and should be chosen based an the optimal total oxygen supply predicted for the wine or grape must in question. The longer the desired storage time, the lower the oxygen level in the container should be and vice versa.

The adjustment of the oxygen level in the container can be performed and monitored by state-of-the-art oxygen management equipment that is principally known to the person skilled in the art.

Methods to precisely measure oxygen levels in a closed container are also known to the person skilled in the art. For example, the Mocon® Ox-tran® method (Mocon Inc., Minneapolis, USA) is widely applied and recommended in different standards such as the ASTM (F1307-02). A very convenient method for measuring oxygen levels according to the present disclosure is by a non-destructive technique known as Nomasense® technology. This method allows measurement of oxygen levels through the container wall by luminescence-based technology using separate sensors supplied by PreSens® (Precision Sensing GmbH, Regensburg, Germany). Detailed description of oxygen measurement technologies and protocols can be found, for example, in Dieval J-B., Vidal S. and Aagaard 0., Packag. Technol. Sci. 2011; DOI: 10.1002/pts.945.

In a further exemplary embodiment of the disclosure, the oxygen level in the container is selected from the oxygen present in the air of the headspace (i.e. the ullage volume between fill level and closure) and the total oxygen present in the container (total package oxygen, TPO). TPO is generally thought of as the sum of dissolved oxygen and the oxygen present in the air of the headspace.

The oxygen level in the container can be influenced by several means. First, contact of the wine with air during filling can result in an increased amount of dissolved oxygen in the wine. Secondly, gaseous oxygen trapped in the container headspace after filling and closure of the container is another major source of oxygen. The amount of oxygen present in the headspace can vary, depending on headspace volume, which is determined by container dimensions, fill level, and/or container neck space that is occupied by the closure, as well as the oxygen concentration in the gas phase occupying the head space. The amount of oxygen present in the gas phase after closing the container can be reduced, for example, by applying headspace management technology such as, for example, evacuation (vacuum) or inerting (e.g. flushing with carbon dioxide or nitrogen) the headspace immediately before the container is closed. Thirdly, after container closure and during storage, oxygen ingress through the closure, as determined by the oxygen transfer rate (OTR) of the closure or the container walls, may be responsible for additional oxygen uptake.

Finally, besides these three aforementioned routes of oxygen uptake, it has been found that immediately after closing wine bottles with natural or synthetic cork stoppers, off-gassing of air from the compressed cork material may further contribute to an initially high local oxygen concentration in the bottle headspace. Such off-gassing of the closure may be caused by the compression which the closure undergoes when being inserted into the bottleneck. The compression may lead to diffusion of air present in the cork in all directions possible, including into the bottle headspace.

The off-gassing phenomenon, which has also been referred to as "desorption" of the closure (Dieval, J.-B. et al., Packag. Technolog. Sci. 2011 and references therein), becomes evident from curves depicting the oxygen ingression kinetics after bottle closure. Without wishing to be bound by theory such curves can generally be divided into two parts. In a first phase, there is relatively fast and non-linear oxygen ingress into the bottle headspace. Later-on, in a second phase, which typically begins a couple of weeks to a year after bottling and lasts for the years of subsequent storage, the oxygen ingress rate is slower but constant and follows a linear curve, the slope of which is defined by the respective closure's OTR.

The first faster and non-linear oxygen ingress is generally caused by the off-gassing of air, which was present in the closure and is forced out of the closure by the compression of the closure in the bottle neck after bottling. The second phase generally is the oxygen that diffuses from the outside atmosphere through the closure and into the bottle headspace. In the following, the gas ingress from within the closure, i.e. the first phase, will be referred to as closure desorption. This is used within the present disclosure synonymous to other suited terms such as off-gassing, outgassing of the closure or ingress of oxygen from within the closure itself upon closing. In particular, the use of the term desorption shall not limit the present disclosure to the physical phenomenon scientifically described as desorption. The term desorption as used in the description of the present disclosure is rather meant to include any release of a gas from the closure itself, which, by way of example, was trapped in the closure, e.g. in voids or cells present in the closure, or dissolved, adsorbed, chemically or otherwise bonded to the closure material and which is released into the interior of the container upon or after closing the container with said closure.

In an exemplary embodiment, the oxygen level in the container is set, maintained and/or varied in a controlled fashion by any of the above described means of influencing the oxygen level in the container.

In an exemplary embodiment, the oxygen level in the container is set, maintained and/or varied in a controlled fashion by closure technology, e.g. by sealing closed the container with a closure having a defined oxygen transfer rate (OTR) and/or a defined amount of closure desorption. In an exemplary embodiment the closure is a synthetic closure having a defined OTR and/or a defined amount of closure desorption. Closures with defined amounts of desorption are described in WO 2013/068473 A1 (corresponds to U.S. Provisional Patent Application Serial No. 61/558,599)

According to another exemplary embodiment of the disclosure, the oxygen level in the container is achieved by supplying a defined starting amount of oxygen to the container interior before sealing closed said container and/or by sealing closed the container with a closure having a defined oxygen transfer rate and/or a defined amount of closure desorption.

According to another exemplary embodiment of the disclosure, the oxygen level in the container is set, maintained and/or varied in a controlled fashion by selecting a container with a defined OTR. In an exemplary embodiment, the container with a defined OTR is a plastic storage or maturation tank or a bag in box. Such containers with defined OTRs are known to the person skilled in the art and commercially available, for example from Flextank Pty Ltd., Australia or from RedOaker, Australia.

According to yet another exemplary embodiment of the disclosure, the oxygen level in the container is set, maintained and/or varied in a controlled fashion by selecting a container that is fitted with built in valves or lids to allow the ingress of defined quantities of oxygen over a certain period of time.

According to a further exemplary embodiment of the disclosure, the oxygen level in the container is set, maintained and/or varied in a controlled fashion by selecting a container that is fitted with equipment allowing the supply of a controlled amount of oxygen into the container interior. In an exemplary embodiment, such an equipment is a micro-oxygenation or a micro-oxydation device. Such devices are known to the person skilled in the art and commercially available, for example from VIVELYS. Micro-oxygenation or micro-oxydation devices function via a progressive and controlled oxygen injection systems. The amount of oxygen to be injected depends on the desired oxygen level that shall be set, maintained and/or varied in the container. According to an exemplary embodiment of this disclosure, the rate of air addition is from 0,1 to 120 ml per liter wine or grape must per month.

Depending on the determined optimal total oxygen supply, especially if the latter is rather low, the oxygen level in the container may also be achieved by depriving the container of a defined amount of oxygen. Advances in headspace management technology such as evacuation or inerting (e.g. flushing with nitrogen) the headspace before closing containers used for wine storage have made it possible to diminish and even eliminate the starting amount of oxygen present in the containers.

In a further embodiment of the disclosure, a method for selecting an optimal closure for storing a wine or a grape must in a container is provided, which comprises the steps of:
a) predicting the oxidability of the wine or grape must that is to be stored according to the electroanalytical method described above; and
b) selecting a closure based on the oxidability predicted in step a), the oxygen transfer rate (OTR) of the closure, the intended length of storage and the desired properties that the wine or grape must shall have upon opening the container after storage.

According to another embodiment the closure selection in step b)is additionally based on the optimal total oxygen supply that can be determined by the method described above.

According to yet another embodiment the closure selection in step b)is additionally based on the amount of closure desorption.

While the closures described herein may, in principle, relate to any kind of closure, due to the special requirements in the wine industry, special closures for wine bottles, barrels or containers such as, for example, a natural or synthetic cork stopper or a screw-cap closure, may be used.

Methods for determining closure OTR are known to the person skilled in the art. For example, mathematical modeling has recently allowed describing the oxygen ingress curve for container closures and their OTR in mathematical terms (Dieval J-B., Vidal S. and Aagaard O., Packag. Technol. Sci. 2011; DOI: 10.1002/pts.945). Closure OTR as used throughout the present disclosure is defined and measured as described in this reference.

The method for selecting an optimal closure disclosed herein enables winemakers to choose - based on the oxidability and the optimal total oxygen supply for the wine or grape must in question - a closure from a range of closures with distinct and consistent OTR values. This tailoring of the wine closure to the specific oxygen requirements of a particular type of wine, may allow wineries to optimize the oxygen-dependent flavor and wine character development for each of their wine product lines and at the same time prevent the formation of unpleasant aromas associated with reduction.

### Examples

Hereinafter, certain exemplary embodiments are described in more detail and specifically with reference to the examples, which, however, are not intended to limit the present disclosure.

Example 1: A set of six wines was analyzed by cyclic voltammetry. The wines were six different white wines produced in 2010 from Grenache blanc grapes harvested in the Languedoc-Roussillon region (France). The voltammograms were recorded using an Edelscan device (EDEL Therapeutics, Switzerland) equipped with disposable multisurface electrode strips.

Between scans the single-use electrode strips were exchanged for new ones and the used electrode strips were discarded. Less than 15 seconds were needed for exchange of electrode strips in between scans. The scans were recorded in a voltage range from 0 to 1200 mV and each scan took 30 seconds.

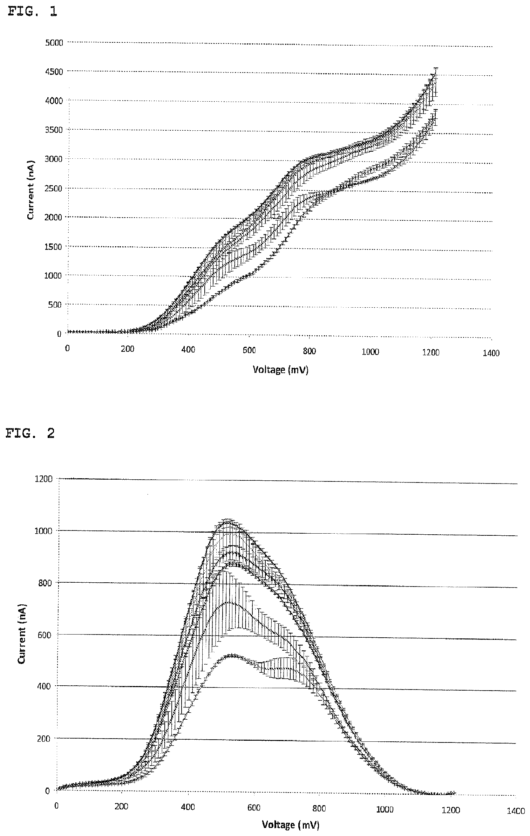

Next to the primary electrochemical curves (voltammograms), which are depicted in Figure 1, the device also computed a processed voltammogram obtained by numerical pseudo-titration of the primary voltammograms according to the mathematical operation described in WO 2006/094529 A1. These processed voltammograms are depicted in Figure 2 and show an approximately bell-shaped curve. The six wines differed in their electrochemical profiles (cf. Figures 1 and 2).

The areas beneath the curves in Figure 2 were calculated by integrating the processed primary current signals over the applied potentials. The result, i.e. the sum of each oxidation current per potential increment is given in table 1 as the antioxidant power (AOP) of the respective wine sample:

**Table 1**

| Wine sample # | Antioxidant Power (AOP) |
|---|---|
| 1 | 672 |
| 2 | 947 |
| 3 | 820 |
| 4 | 867 |
| 5 | 907 |
| 6 | 520 |

Example 2: To determine experimentally the oxidability of each wine analyzed voltammetrically in example 1, an accelerated aging test simulating wine aging was performed.

For the accelerated aging test, 10 mL of each wine was placed in a 40 ml bottle and left open to react with air for 14 days at 25°C. Oxidability of each sample was then determined by the difference between absorbance at 420 nm before and after the accelerated aging test. All experiments were performed in triplicate. Absorbance at 420 nm is an indicator of wine browning, which is one of the major modifications induced by oxidation.

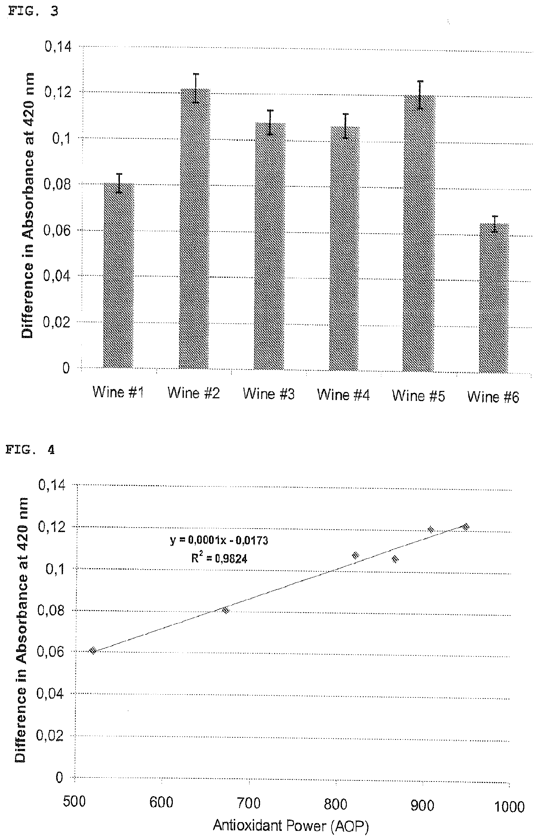

The results of the accelerated aging test are given in table 2 below and are depicted as bar chart in Figure 3. The six wines showed different oxidability values, with samples 1 and 6 showing relatively low oxidability.

**Table 2**

| Wine sample # | Difference in |
|---|---|
| 1 | 0,0806 |
| 2 | 0,1222 |
| 3 | 0,1078 |
| 4 | 0,1064 |
| 5 | 0,1206 |
| 6 | 0,0649 |

Example 3: The AOP values listed in table 1 (cf. example 1) were plotted against the experimentally determined wine oxidability values listed in table 2 (cf. example 2 and Figure 3). A linear regression analysis was performed to determine the R² value. The resulting correlation plot is depicted in Figure 4.

A very high degree of correlation was observed (R² = 0.982), indicating that the electrochemical data, and in particular the processed voltammograms and the AOP value, have great potential to predict wine oxidability.

## Claims

1. An electroanalytical method for predicting the oxidability of a wine or a grape must, comprising the steps of
(a) recording an electrochemical signal of a sample of the wine or grape must;
(b) comparing an electrochemical signature of the electrochemical signal obtained in step a) with reference electrochemical signatures obtained from wines or grape musts with known oxidability; and
(c) predicting the oxidability of the sample tested based on the comparison performed in step b), wherein
the electrochemical signal is a processed electrochemical signal in which the primary signal is modulated by applying thereto a mathematical treatment based on a virtual electrochemical titration;
wherein the comparison of the electrochemical signatures in step b) is performed by comparing the area beneath the curve of the processed electrochemical signal.

2. The electroanalytical method according to claim 1, wherein the mathematical treatment is based on a Fermi-Dirac function which simulates a virtual electrochemical titration of a reference molecule which oxidation potential ranges from 0 to 1.5 V and which includes any monotonous decreasing dimensionless function between one and zero.

3. The electroanalytical method according to any one of claims 1 or 2, wherein said area beneath the curve is defined as an antioxidant power, expressed in electrical power units or in specific units such as in antioxidant power units, by integrating the modulated primary current signal over the applied potential.

4. The electroanalytical method according to any one of the preceding claims, wherein the primary electrochemical signal is a voltammogram.

5. The electroanalytical method according to claim 4, wherein the voltammogram is a cyclic voltammogram or a sweep voltammogram.

6. The electroanalytical method according to any one of the preceding claims, wherein the electrochemical signal is recorded using a device comprising a multiple electrode system with at least one working electrode, one reference electrode and one auxiliary electrode.

7. The electroanalytical method according to any one of the preceding claims, wherein the electrochemical signal is recorded using a disposable electrode.

8. Use of an electrochemical signal recorded for a wine or a grape must as marker for predicting the oxidability of the wine or grape must in a method according to any one of claims 1 to 7.

9. A method for predicting the optimal total oxygen supply for storing a wine or a grape must in a container comprising the steps of
(a) predicting the oxidability of the wine or grape must according to the method described in any one of claims 1 to 7;
(b) predicting the optimal total oxygen supply based on the oxidability predicted in step a) and the desired properties that the wine shall have after storage.

10. A method for wine maturation and/or ageing comprising the steps of
(a) predicting the optimal total oxygen supply of the wine or grape must according to the method of claim 9
(b) storing the wine or grape must in a container over a defined period of time, wherein the oxygen level in the container and the storage time are adjusted so that the optimal total oxygen supply as determined in step a) is achieved at the end of the storage time.

11. Method according to claim 10, wherein the oxygen level in the container is achieved by supplying a defined starting amount of oxygen to the container interior before sealing closed said container and/or by sealing closed the container with a closure having a defined oxygen transfer rate and/or a defined amount of closure desorption.

12. Method according to any one of claims 10 or 11, wherein the container is selected from the group consisting of barrel, tank, bottle, canister, jerry can and plastic bag.

13. A method for selecting an optimal closure for storing a wine or a grape must in a container comprising the steps of
(a) predicting the oxidability of the wine or grape must that is to be stored according to the method described in any one of claims 1 to 7; and
(b) selecting a closure based on the oxidability predicted in step a), the oxygen transfer rate of the closure, the intended length of storage and the desired properties that the wine or grape must shall have upon opening the container after storage.

## Patentansprüche

1. Elektroanalytisches Verfahren zum Vorhersagen der Oxidierbarkeit eines Weins oder eines Traubenmosts, das die folgenden Schritte umfasst:
(a) Aufzeichnen eines elektrochemischen Signals einer Probe des Weins oder des Traubenmosts;
(b) Vergleichen einer elektrochemischen Signatur des in Schritt a) erhaltenen elektrochemischen Signals mit elektrochemischen Referenzsignaturen, die von Weinen oder Traubenmosten mit bekannter Oxidierbarkeit erhalten werden; und
(c) Vorhersagen der Oxidierbarkeit der geprüften Probe basierend auf dem in Schritt b) durchgeführten Vergleich, wobei
das elektrochemische Signal ein verarbeitetes elektrochemisches Signal ist, in dem das primäre Signal durch Anwenden einer mathematischen Behandlung darauf, die auf einer virtuellen elektrochemischen Titration basiert, moduliert wird;
wobei der Vergleich der elektrochemischen Signaturen in Schritt b) durch Vergleichen des Bereichs unter der Kurve des verarbeiteten elektrochemischen Signals durchgeführt wird.

2. Elektroanalytisches Verfahren nach Anspruch 1, wobei die mathematische Behandlung auf einer Fermi-Dirac-Funktion basiert, die eine virtuelle elektrochemische Titration eines Referenzmoleküls simuliert, dessen Oxidationspotential von 0 bis 1,5 V reicht und die eine beliebige monotone abnehmende dimensionslose Funktion zwischen Eins und Null beinhaltet.

3. Elektroanalytisches Verfahren nach einem der Ansprüche 1 oder 2, wobei der Bereich unter der Kurve durch Integrieren des modulierten primären Stromsignals über das angewandte Potential als eine antioxidative Leistung definiert ist, die in Einheiten elektrischer Leistung oder in spezifischen Einheiten, wie etwa in Einheiten antioxidativer Leistung ausgedrückt wird.

4. Elektroanalytisches Verfahren nach einem der vorhergehenden Ansprüche, wobei das primäre elektrochemische Signal ein Voltammogramm ist.

5. Elektroanalytisches Verfahren nach Anspruch 4, wobei das Voltammogramm ein Cyclovoltammogramm oder ein Sweep-Voltammogramm ist.

6. Elektroanalytisches Verfahren nach einem der vorhergehenden Ansprüche, wobei das elektrochemische Signal unter Verwendung einer Vorrichtung aufgezeichnet wird, die ein Mehrfachelektrodensystem mit wenigstens einer Arbeitselektrode, einer Referenzelektrode und einer Hilfselektrode umfasst.

7. Elektroanalytisches Verfahren nach einem der vorhergehenden Ansprüche, wobei das elektrochemische Signal unter Verwendung einer Einwegelektrode aufgezeichnet wird.

8. Verwendung eines elektrochemischen Signals, das für einen Wein oder einen Traubenmost als Marker zum Vorhersagen der Oxidierbarkeit des Weins oder des Traubenmosts aufgezeichnet wird, in einem Verfahren nach einem der Ansprüche 1 bis 7.

9. Verfahren zum Vorhersagen der optimalen Gesamtsauerstoffzufuhr zum Lagern eines Weins oder eines Traubenmosts in einem Gefäß, das die folgenden Schritte umfasst:
(a) Vorhersagen der Oxidierbarkeit des Weins oder des Traubenmosts nach dem in einem der Ansprüche 1 bis 7 beschriebenen Verfahren;
(b) Vorhersagen der optimalen Gesamtsauerstoffzufuhr basierend auf der in Schritt a) vorhergesagten Oxidierbarkeit und der gewünschten Eigenschaften, die der Wein nach einer Lagerung aufweisen soll.

10. Verfahren für eine Weinreifung und/oder eine Weinalterung, das die folgenden Schritte umfasst:
(a) Vorhersagen der optimalen Gesamtsauerstoffzufuhr des Weins oder des Traubenmosts nach dem Verfahren nach Anspruch 9,
(b) Lagern des Weins oder des Traubenmosts in einem Gefäß über eine definierte Zeitspanne, wobei der Sauerstoffgehalt in dem Gefäß und die Lagerungszeit angepasst werden, so dass an dem Ende der Lagerungszeit die in Schritt a) bestimmte optimale Gesamtsauerstoffzufuhr erreicht wird.

11. Verfahren nach Anspruch 10, wobei der Sauerstoffgehalt in dem Gefäß durch Bereitstellen einer definierten Ausgangsmenge an Sauerstoff in dem Gefäßinnenraum vor einem abgedichteten Verschließen des Gefäßes und/oder durch das abgedichtete Verschließen des Gefäßes mit einem Verschluss erreicht wird, der eine definierte Sauerstoffübertragungsrate und/oder eine definierte Menge an Verschlussdesorption aufweist.

12. Verfahren nach einem der Ansprüche 10 oder 11, wobei das Gefäß aus der Gruppe ausgewählt ist, die aus Fass, Tank, Flasche, Behälter, Kanister und Plastikbeutel besteht.

13. Verfahren zum Auswählen eines optimalen Verschlusses zum Lagern eines Weins oder eines Traubenmosts in einem Gefäß, das die folgenden Schritte umfasst:
(a) Vorhersagen der Oxidierbarkeit des Weins oder des Traubenmosts, der nach dem in einem der Ansprüche 1 bis 7 beschriebenen Verfahren gelagert werden soll; und
(b) Auswählen eines Verschlusses basierend auf der in Schritt a) vorhergesagten Oxidierbarkeit, der Sauerstoffübertragungsrate des Verschlusses, der vorgesehenen Dauer der Lagerung und den gewünschten Eigenschaften, die der Wein oder der Traubenmost bei einem Öffnen des Gefäßes nach der Lagerung aufweisen soll.

## Revendications

1. Procédé électroanalytique pour prédire l'oxydabilité d'un vin ou d'un moût de raisin, comprenant les étapes consistant à :
(a) enregistrer un signal électrochimique d'un échantillon de vin ou de moût de raisin ;
(b) comparer une signature électrochimique du signal électrochimique obtenue à l'étape a) avec des signatures électrochimiques de référence obtenues à partir de vins ou de moûts de raisin d'oxydabilité connue ; et
(c) prédire l'oxydabilité de l'échantillon testé sur la base de la comparaison effectuée à l'étape b), dans lequel
le signal électrochimique est un signal électrochimique traité dans lequel le signal primaire est modulé en lui appliquant un traitement mathématique basé sur un titrage électrochimique virtuel ;
dans lequel la comparaison des signatures électrochimiques à l'étape b) est effectuée en comparant la zone sous la courbe du signal électrochimique traité.

2. Procédé électroanalytique selon la revendication 1, dans lequel le traitement mathématique est basé sur une fonction de Fermi-Dirac qui simule un titrage électrochimique virtuel d'une molécule de référence dont le potentiel d'oxydation va de 0 à 1,5 V et qui comporte chacune fonction sans dimension décroissante monotone entre un et zéro.

3. Procédé électroanalytique selon l'une quelconque des revendications 1 ou 2, dans lequel ladite zone sous la courbe est définie en tant qu'un pouvoir antioxydant, exprimé en unités de pouvoir d'énergie électrique ou en unités spécifiques telles que des unités de pouvoir antioxydant, en intégrant le signal de courant primaire modulé sur le potentiel appliqué.

4. Procédé électroanalytique selon l'une quelconque des revendications précédentes, dans lequel le signal électrochimique primaire est un voltamogramme.

5. Procédé électroanalytique selon la revendication 4, dans lequel le voltamogramme est un voltamogramme cyclique ou un voltamogramme à balayage.

6. Procédé électroanalytique selon l'une quelconque des revendications précédentes, dans lequel le signal électrochimique est enregistré à l'aide d'un dispositif comprenant un système à électrodes multiples doté d'au moins une électrode de travail, une électrode de référence et une électrode auxiliaire.

7. Procédé électroanalytique selon l'une quelconque des revendications précédentes, dans lequel le signal électrochimique est enregistré à l'aide d'une électrode jetable.

8. Utilisation d'un signal électrochimique enregistré pour un vin ou un moût de raisin en tant que marqueur pour prédire l'oxydabilité du vin ou du moût de raisin dans un procédé selon l'une quelconque des revendications 1 à 7.

9. Procédé de prédiction d'apport d'oxygène total optimal pour stocker un vin ou un moût de raisin dans un récipient comprenant les étapes consistant à :
(a) prédire l'oxydabilité du vin ou du moût de raisin selon le procédé décrit dans l'une quelconque des revendications 1 à 7 ;
(b) prédire l'apport d'oxygène total optimal sur la base de l'oxydabilité prédite à l'étape a) et les propriétés souhaitées du vin après stockage.

10. Procédé de maturation et/ou de vieillissement du vin comprenant les étapes consistant à :
(a) prédire l'apport d'oxygène total optimal du vin ou du moût de raisin selon le procédé de la revendication 9
(b) stocker le vin ou le moût de raisin dans un récipient pendant une période définie, le niveau d'oxygène dans le récipient et la durée de stockage étant réglés de telle sorte que l'apport d'oxygène total optimal tel que déterminé à l'étape a) est atteint à la fin de la durée de stockage.

11. Procédé selon la revendication 10, dans lequel le niveau d'oxygène dans le récipient est atteint en fournissant une quantité d'oxygène de départ définie à l'intérieur du récipient avant de fermer hermétiquement ledit récipient et/ou en fermant hermétiquement le récipient au moyen d'une fermeture ayant une vitesse de transfert d'oxygène définie et/ou une quantité définie de désorption de fermeture.

12. Procédé selon l'une quelconque des revendications 10 ou 11, dans lequel le récipient est choisi dans le groupe constitué par un fût, un réservoir, une bouteille, un bidon, un jerricane et un sac en plastique.

13. Procédé de sélection d'une fermeture optimale pour stocker un vin ou un moût de raisin dans un récipient comprenant les étapes consistant à :
(a) prédire l'oxydabilité du vin ou du moût de raisin à stocker selon le procédé décrit dans l'une quelconque des revendications 1 à 7 ; et
(b) sélectionner une fermeture sur la base de l'oxydabilité prédite à l'étape a), de la vitesse de transfert d'oxygène de la fermeture, de la longueur de stockage prévue et des propriétés souhaitées du vin ou du moût de raisin lors de l'ouverture du récipient après stockage.
